# EUROPÄISCHE PATENTANMELDUNG

(11) **EP 2 614 798 A1**
(43) Veröffentlichungstag der Anmeldung: **17.07.2013**
(21) Anmeldenummer: 13000109.2
(22) Anmeldetag: 10.01.2013
(51) Int. Cl.: A61F 5/56, A61F 5/58, A63B 23/03

(54) **Therapiegerät zur myofunktionalen Therapie**

(30) Priorität: 10.01.2012 DE 102012000287
(71) Anmelder: Weber, Joachim, 67071 Ludwigshafen (DE)
(72) Erfinder: Weber, Joachim, 67071 Ludwigshafen (DE)
(74) Vertreter: Roth, Klaus

(57) **Zusammenfassung**

Es wird ein Therapiegerät zur myofunktionalen Therapie mit einem Mundeinsatz, an dem wenigstens ein Funktionselement angebracht ist, vorgeschlagen, mittels dem eine verbesserte Therapie zielgerichtet auf das zur therapierende Individuum sowie dessen Dysfunktion möglich ist. Dies wird erfindungsgemäß dadurch erreicht, dass das Funktionselement als Zungen-Funktionselement zum Therapieren und/oder Trainieren zumindest der Zunge bzw. der Zungenmuskulatur ausgebildet ist.

## Beschreibung

Die Erfindung betrifft ein Therapiegerät zur myofunktionalen Therapie nach dem Oberbegriff des Anspruchs 1.

Mit der EP 1 516 604 B1 ist ein intraorales Therapiegerät zur Schnarchtherapie bekannt geworden. Dieses Therapiegerät umfasst eine Oberkieferschiene und eine Unterkieferschiene, die über wenigstens einen Stab miteinander verbunden sind. Ein solches Therapiegerät ist zur myofunktionalen Therapie nicht geeignet.

Mit der DE 11 2008 002 157 T5 ist eine Zahnschale bekannt geworden, mittels der Einbleichverfahren durchführbar ist. Auch eine solche Zahnschale ist nicht zur myofunktionalen Therapie geeignet.

Mit der DE 19 844 628 ist ein kieferorthopädisches Therapiegerät insbesondere zur Behandlung atembezogener Schlafstörungen bekannt geworden. Ein solches Gerät ist insbesondere auch als Kauschiene zum Schutz der Zähne vor Gewalteinwirkung im Gebrauch, dient jedoch nicht der myofunktionalen Therapie.

Mit der DE 19 903 937 ist ein Therapiegerät für die Logopädie zur Stimulierung von Hautbereichen bekannt, wobei ein Wirkteil von einem Antriebsteil in Vibrationsbewegung versetzt wird und unter anderem auch ein Einsatz im Mund des Patienten vorgesehen ist.

Mit der DE 10 2007 009 698 A1 ist ein Mundschutz bekannt geworden, der durch Maßnahmen zur genauen Einhaltung der idealen Bissposition eine Leistungssteigerung bewirken soll.

Alle die genannten Vorrichtungen sind zur myofunktionalen Therapie nicht geeignet.

Mit der DE 20 2006 011 831 U1 ist ein Mundfunktionsübungsgerät bekannt geworden, dass im gesamten Mundvorhof, dass heißt im Wesentlichen in dem Raum zwischen den Zähnen und den Lippen oder den Wangen zum Einsatz kommt.

Mit der DE 20 2006 011 829 U1 wiederum ist ein therapeutisches Spielzeug zur Systemfunktionstherapie bekannt, dass eine spielerische Durchführung von Schluck und Saugübungen dadurch ermöglicht, dass eine kindgerechte Maske oder Figur über die Maul- Mund- oder Rüsselöffnung mit dem okklusalen Raum gekoppelt ist.

Ausgehend von derartigen Vorrichtungen hat die Erfindung die Aufgabe, ein Therapiegerät zur myofunktionalen Therapie vorzuschlagen, mittels dem eine verbesserte Therapie zielgerichtet auf das zur therapierende Individuum sowie dessen Dysfunktion möglich ist.

Diese Aufgabe wird ausgehend von einem Therapiegerät nach dem Oberbegriff des Anspruchs 1 durch dessen kennzeichnende Merkmale gelöst.

Dementsprechend zeichnet sich ein erfindungsgemäßes Therapiegerät dadurch aus, dass das Funktionselement als Zungen-Funktionselement zum Therapieren und/oder Trainieren zumindest der Zunge bzw. der Zungenmuskulatur ausgebildet ist.

Mit Hilfe der Erfindung kann vor allem die Zunge bzw. deren Muskulatur zielgerichtet trainiert bzw. therapiert werden. Hierbei wird zugleich auch die zur Zungenmuskulatur benachbarte Muskulatur mit einbezogen bzw. trainiert und therapiert. Dies eröffnet völlig neuartige Möglichkeiten der individuellen Therapie bzw. des individuellen Trainings. Vorteilhafterweise kann eine Kombination eines erfindungsgemäßen Zungen-Funktionselement mit einem Funktionselement für die Lippen und/oder den Mundvorhof etc. vorgesehen werden. Beispielsweise kann der Mundeinsatz auch ein Lippen-Funktionselement zum Therapieren und/oder Trainieren zumindest der Lippen umfassen bzw. haltern. Hiermit kann eine ganzheitliche bzw. vollständige Therapie bzw. Training und/oder eine Individualisierung des erfindungsgemäßen Therapiegerätes erreicht werden, wie dies bislang nicht möglich war. Demzufolge können gemäß der Erfindung individuelle Dysfunkionen zielgerichteter abgegangen bzw. beseitigt werden.

Vorzugsweise sind zwischen einem Mundeinsatz und einem Zungen-Funktionselement lösbare Verbindungselemente vorgesehen, sodass wenigstens ein Zungen-Funktionselement austauschbar ist. Auf diese Weise kann das Therapiegerät an die zu therapierende Person angepasst werden. Eine Anpassung kann beispielsweise an die Anatomie oder an das Alter der Person oder aber auch an das Störungsbild oder sonstige Erfordernisse erfolgen. Hiermit wird eine sehr große Flexibilität erreicht. Auch können z.B. im Laufe der Therapie bzw. des Trainings die Zungen-Funktionselemente bzw. die Funktionselemente ohne großen Aufwand ausgetauscht und/oder an die jeweilige Therapie-/Trainings-Phase bzw. den individuellen Fortschritt optimal angepasst werden.

Vorzugsweise wird der Mundeinsatz so ausgestaltet, dass er ein oder mehrere Zähne der zu therapierenden Personen wenigstens teilweise umschließt. Auf diese Weise wird eine zuverlässige Fixierung des Therapie- oder Trainingsgeräts im Mund gewährleistet. In Frage kommen hierbei angeformte Einsätze, wie sie aus der Kieferorthopädie oder der zahntechnischen Behandlung bekannt sind,

Insbesondere kann der Mundeinsatz ähnlich wie bekannte Mundeinsätze für andere Zwecke ausgestaltet werden, beispielsweise als sogenannter Aqualizer und/oder als Kauschiene und/oder als Mundschutz. Für alle diese Anwendungszwecke sind Mundeinsätze bekannt, die individuell dem Gebiss des Trägers angepasst bzw. angeformt werden.

Vorteilhafterweise ist der Mundeinsatz an ein oder mehrere Zähne angeformt. Die genaue Anformung an das Gebiss des Trägers erlaubt eine problemlose dauerhafte Anwendung des Therapiegeräts über einen längeren Zeitraum.

Vorzugsweise werden als Zungen-Funktionselement Objekte vorgesehen, die die Zungenbewegung stimulieren. In Frage kommen beispielsweise ein Riemen, ein oder mehrere Körper, wie Ringe, Kugeln, vieleckige Körper, beispielsweise Dreieckskörper, Quaderkörper und/oder ein oder mehrere Bürsten. Auch der Einsatz von sogenannten Zungenexpandern oder elastischen Bändern ist möglich, um insbesondere die Bewegung, Lage und Spannung der Zunge zu trainieren.

Weiterhin können bei einem erfindungsgemäßen Therapiegerät als Zungen-Funktionselement auch eine oder mehrere Halterungen für sich auflösende oder verzehrbare Übungselemente vorgesehen werden. So kann beispielsweise eine Halterung für einen Geschmacksträger wie ein Fruchtgummi, Lutschbonbons oder dergleichen vorgesehen werden. Auch durch derartige Gegenstände kann das Mund- oder Zungentraining gefördert werden. Insbesondere bei Kindern kann mittels derartiger Elemente auch die Trainingsmotivation erheblich gesteigert werden.

Weiterhin kommen als Zungen-Funktionselement auch Saugelemente wie Saugröhrchen in Frage, an denen verschiedene Trainingseffekte für die Zungen und/oder Gesichtsmuskulatur möglich sind. Derartige Saugelemente können beispielsweise auch noch mit Zungendruckventilen versehen werden, um das Zungen- und Gesichtsmuskeltraining komplexer zu gestalten.

Auch bewegliche Trainingskörper wie beispielsweise bewegliche Kugeln oder sonstige o.a. Körper können als Zungen-Funktionselemente sinnvoll eingesetzt werden.

Die Verbindungselemente sind nach Möglichkeit so ausgestaltet, dass ein einfacher Wechsel des Zungen-Funktionselementes möglich ist. So kann beispielsweise ein Austausch über eine Druckknopfverbindung oder allgemeiner ausgedrückt über eine Rast- und/oder Schnappverbindung vorgesehen werden.

Auch Verriegelungsmechanismen, beispielsweise Bajonettverschlüsse oder Drehschwenkmechanismen sind beispielweise mit oder ohne Kombination von Schnapp- oder Rastverbindungen denkbar. Wesentlich ist in jedem Fall die einfach und nach Möglichkeit werkzeuglose Auswechslung des Zungen-Funktionselements.

Ein erfindungsgemäßes Therapiegerät ist in der Zeichnung dargestellt und wird anhand der Figuren nachfolgend näher erläutert.

Im Einzelnen zeigen
- Figur 1: eine schematische perspektivische Darstellung eines interoralen Therapiegeräts mit Verbindungselementen.
- Figur 2: verschieden Zungen-Funktionselemente, die als Wechselaufsätze für einen Therapiegerät gemäß Figur 1 in Frage kommen.
- Figur 3: weitere Zungen-Funktionselemente für ein Therapiegerät.

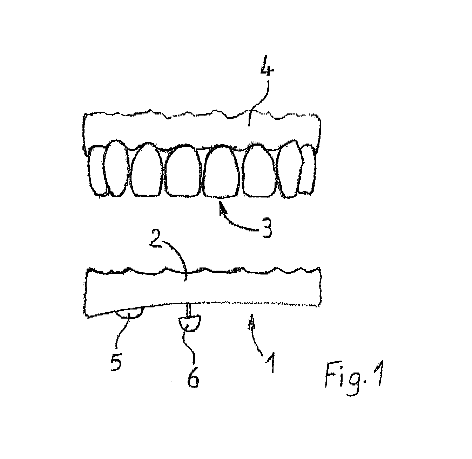

Im Einzelnen zeigen Figur 1 ein erfindungsgemäßes Therapiegerät 1 mit einem Mundeinsatz 2, der nach Art einer Kauschiene ausgebildet ist, sodass er formschlüssig auf die in Figur 1 ebenfalls dargestellten Zähne 3 des Oberkiefers 4 eines Patienten passt. Am Mundeinsatz 2 sind schematisch verschiedene Elemente 5, 6 dargestellt, die das Anbringen von Verbindungselementen veranschaulichen sollen. Die Form des Verbindungselementes 6 als abstehender Fortsatz mit einer halbkugelförmigen Endung kann beispielsweise für eine Rastverbindung mit einem entsprechenden Gegenstück dienen. Es ergibt sich hierdurch eine druckknopfartige Rastverbindung.

Das Verbindungselement 5 kann beispielsweise wie das Gegenstück einer solchen druckknopfartigen Verbindung als hohe Schale oder dergleichen ausgebildet sein, in die dann das Gegenstück eines Zungen-Funktionselementes einrasten kann.

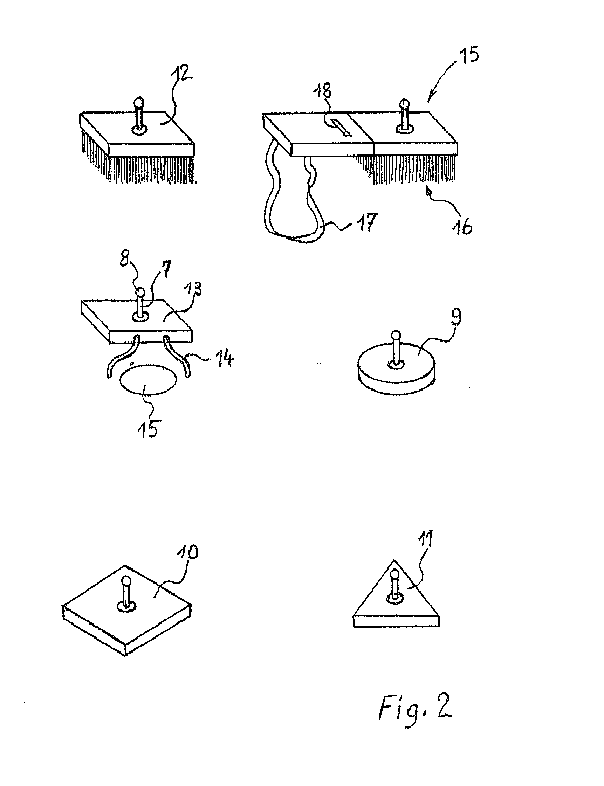

In Figur 2 sind verschiedene Zungen-Funktionselemente als Wechselaufsätze für einen Mundeinsatz 2 gemäß Figur 1 dargestellt. Alle diese Funktionseinsätze tragen in dem dargestellten Ausführungsbeispiel einen Fortsatz 10 mit einer endseitigen Rastkugel 8, die in eine druckknopfartige Rastverbindung passt. Figur 2 zeigt verschiedenartige Zungen-Funktionselemente, die alternativ oder gemeinsam zum Einsatz kommen können. Insbesondere kann als Zungen-Funktionselement ein Formteil wie beispielsweise eine Scheibe 9, ein Dreieckskörper 10, ein Quaderkörper 11 ein Bürsteneinsatz 12 zum Einsatz kommen. Auch ein Halterungskörper 13, beispielsweise mit Haltespangen 14 zum Fixieren eines Geschmacksträgers wie ein Fruchtgummi 15 kann erfindungsgemäß zum Einsatz kommen.

Ein Zungen-Funktionselement kann dabei auch mehrere Ausgestaltungen für unterschiedliche Trainingsfunktionen aufweisen. So kann beispielsweise das Zungen-Funktionselement 15 ein Bürstenbereich 16, ein elastisches Band 17, beispielsweise ein Gummiband, sowie ein Halteschlitz 18 für ein Geschmacksträger oder ein anderes mechanisches Trainingselement aufweisen.

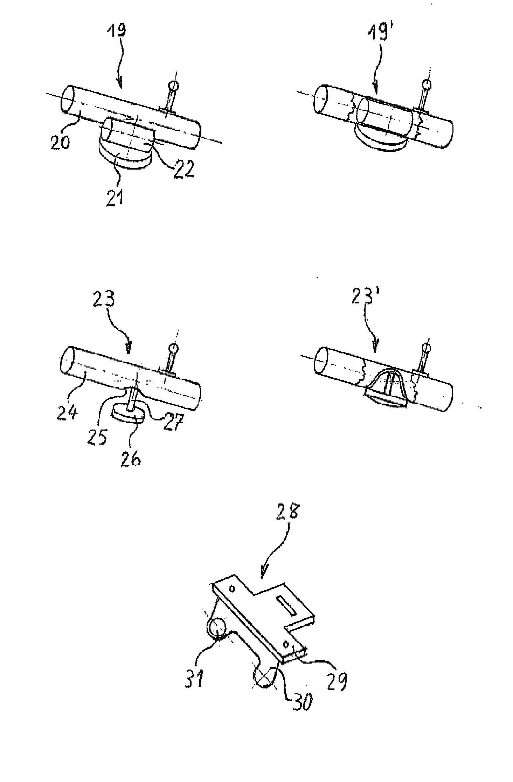

In Figur 3 sind weitere Zungen-Funktionselemente schematisch dargestellt, deren Verbindungselemente den Zungen-Funktionselementen gemäß Figur 2 entsprechen.

So zeigt Figur 3 beispielsweise ein Saugtrainingsgerät 19 mit einem Saugrohr 20, das einen Zungenstößel 21 mit Schließkörper 22 aufweist. Durch das Saugtrainingsgerät 19 ist der offene Zustand des Saugrohrs 20 dargestellt, während mit dem Saugtrainingsgerät 19' der eingedrückte geschlossene Zustand dargestellt ist, bei dem über den Zungenstößel 21 der Schließkörper 22 in das Saugrohr 20 eingedrückt ist. Der Zungenstößel 21 mit dem Schließkörper 22 bildet somit ein Zungendruckventil, das zum Zungentraining bzw. auch zum Mund-oder Gesichtsmuskeltraining über die Zunge geöffnet und geschlossen werden kann.

In entsprechender Weise kann ein Trainingselement 23 ein Blasrohr 24 umfassen, dass eine Öffnung 25 umfasst, die mittels eines Ventilkörpers 26 ganz oder teilweise verschließbar ist. Der Ventilkörper 26 kann über einen Stößel 27 im Blasrohr 24 gehaltert und geführt sein.

Weiterhin ist in Figur 3 ein Zungen-Funktionselement 28 dargestellt, bei dem ein Haltekörper 29 einen Lagerbügel 30 trägt. In dem Lagerbügel 30 sind ein oder mehrere Kugeln 31 beweglich gelagert. Die Kugel 31 kann dabei sowohl translatorisch als auch rotatorisch beweglich gelagert sein. Die Trainingskugel kann somit verschoben oder gedreht werden. Insbesondere kann an dieser Trainingskugel auch zu Trainingszwecken gesaugt werden.

Neben den dargestellten Zungen-Funktionselementen sind eine Vielzahl weiterer Zungen-Funktionselemente einzeln oder in Kombination miteinander erfindungsgemäß verwendbar. Dabei können mehrere Zungen-Funktionselemente auf einem Trägerkörper gemeinsam an dem Mundeinsatz befestigt werden oder aber der Mundeinsatz kann mit verschiedenen Verbindungselementen versehen werden, sodass verschiedene separate Zungen-Funktionselemente verbindbar sind.

Wesentlich bei der Erfindung ist die flexible Anpassbarkeit an unterschiedliche Gegebenheiten der zu trainierenden Person, beispielsweise psychologische Besonderheiten, individuelle Besonderheiten wie Alter, oder auch die Anpassbarkeit an unterschiedliche Störungsbilder, sogenannte Dysfunktionen.

### Bezugszeichenliste:

- 1: Therapiegerät
- 2: Mundeinsatz
- 3: Zähne
- 4: Oberkiefer
- 5: Element
- 6: Element
- 7: Fortsatz
- 8: Rastkugel
- 9: Kreisscheibe
- 10: Dreieckskörper
- 11: Quaderkörper
- 12: Bürsteneinsatz
- 13: Halterungskörper
- 14: Haltespange
- 15: Zungen-Funktionselement
- 16: Bürstenbereich
- 17: elastisches Band
- 18: Halteschlitz
- 19: Saugtrainingsgerät
- 20: Saugrohr
- 21: Zungenstößel
- 22: Schließkörper
- 23: Trainingselement
- 24: Blasrohr
- 25: Öffnung
- 26: Ventilkörper
- 27: Stößel
- 28: Zungen-Funktionselement
- 29: Haltekörper
- 30: Lagerbügel
- 31: Kugel

## Patentansprüche

1. Therapiegerät zur myofunktionalen Therapie mit einem Mundeinsatz, an dem wenigstens ein Funktionselement angebracht ist, **dadurch gekennzeichnet, dass** das Funktionselement als Zungen-Funktionselement zum Therapieren und/oder Trainieren zumindest der Zunge bzw. der Zungenmuskulatur ausgebildet ist.

2. Therapiegerät nach Anspruch 1, **dadurch gekennzeichnet, dass** das Zungen-Funktionselement und der Mundeinsatz lösbarer Verbindungselemente aufweisen, sodass das Zungen-Funktionselement austauschbar ist.

3. Therapiegerät nach einem der vorgenannten Ansprüche, **dadurch gekennzeichnet, dass** der Mundeinsatz als Equaliser und/oder Kauschiene und/oder Mundschutz ausgebildet ist.

4. Trainingsgerät nach einem der vorgenannten Ansprüche, **dadurch gekennzeichnet, dass** als Zungen-Funktionselement eine Bürste und/oder ein Zungenexpander und/oder ein elastisches Band vorgesehen ist.

5. Trainingsgerät nach einem der vorgenannten Ansprüche, **dadurch gekennzeichnet, dass** als Zungen-Funktionselement eine Kreisscheibe und/oder einen Ring und/oder ein vieleckiger Körper vorgesehen ist.

6. Trainingsgerät nach einem der vorgenannten Ansprüche **dadurch gekennzeichnet, dass** als Zungen-Funktionselement eine Halterung für verzehrbare Übungsteile, insbesondere für Geschmacksträger, vorgesehen ist.

7. Therapiegerät nach einem der vorgenannten Ansprüche, **dadurch gekennzeichnet, dass** als Zungen-Funktionselement ein Saugelement wie ein Saugrohr und/oder Saugkugeln vorgesehen ist.

8. Therapiegerät nach einem der vorgenannten Ansprüche, **dadurch gekennzeichnet, dass** ein Blaselement wie ein Blasrohr vorgesehen ist.

9. Therapiegerät nach einem der vorgenannten Ansprüche, **dadurch gekennzeichnet, dass** als Zungen-Funktionselement ein beweglicher Trainingskörper vorgesehen ist.

10. Therapiegerät nach einem der vorgenannten Ansprüche, **dadurch gekennzeichnet, dass** das/die Verbindungselemente als Rast- und/oder Schnappelemente und/oder Riegelelemente ausgebildet ist/sind.
